# EUROPÄISCHE PATENTSCHRIFT

(11) **EP 2 340 815 B1**
(45) Veröffentlichungstag und Bekanntmachung des Hinweises auf die Patenterteilung: **22.08.2012**
(21) Anmeldenummer: 10196347.8
(22) Anmeldetag: 21.12.2010
(51) Int. Cl.: A61K 9/70, A61F 13/02, B65H 39/16, C09J 7/02, B65H 35/02

(54) **Verfahren und Vorrichtung zur Herstellung einer überlappenden Schutzfolie für ein transdermales therapeutisches System**
Method and device for producing an overlapping protective film for a transdermal therapeutic system
Procédé et dispositif de fabrication d'une feuille de protection se chevauchant pour un système thérapeutique transdermique

(30) Priorität: 04.01.2010 DE 102010000661
(43) Veröffentlichungstag der Anmeldung: 06.07.2011
(73) Patentinhaber: Acino AG, 83714 Miesbach (DE)
(72) Erfinder: Dabdoub, Nicola, 80933, München (DE); Müller, Michael, 83703, Gmund (DE)
(74) Vertreter: Beetz & Partner

(56) Entgegenhaltungen:
- EP-A1- 1 967 171
- WO-A1-91/17048
- WO-A1-92/10154
- WO-A1-92/17237
- DE-A1- 19 925 338
- FR-A1- 2 776 518
- US-A- 3 399 884
- US-A- 6 120 792
- US-A1- 2003 164 103
- US-A1- 2003 219 297
- US-B1- 6 280 764
- US-B1- 6 298 529

## Beschreibung

Die vorliegende Erfindung bezieht sich auf transdermale therapeutische Systeme, die eine Schutzfolie aufweisen, die zweigeteilt ist und sich überlappt, sowie Verfahren für ihre Herstellung.

Transdermale therapeutische Systeme dienen in der Regel dazu, Wirkstoffe zu verabreichen, indem sie auf der Haut appliziert werden und der enthaltene Wirkstoff an die Haut abgegeben wird. Sie umfassen gewöhnlich ein Reservoir für den Wirkstoff, eine wirkstoffundurchlässige Rückschicht sowie eine Schutzfolie, die vor der Applikation des Systems entfernt wird. Grundsätzlich können zwei verschiedene Arten von Systemen unterschieden werden, nämlich die so genannten Matrixsysteme, bei denen der Wirkstoff in einer Polymermatrix enthalten ist, die zumeist aus einem selbsthaftenden druckempfindlichen Polymer gebildet ist, und die so genannten Reservoirsysteme, die aus einem den Wirkstoff enthaltenden flüssigen, halbfesten oder festen Reservoir und einer die Wirkstoffabgabe regulierenden Membran bestehen.

Beim Aufbringen des transdermalen therapeutischen Systems, das im Folgenden auch als Pflaster bezeichnet wird, wird die Schutzfolie von dem System entfernt und das Pflaster wird auf der Haut appliziert. Um die Schutzfolie in einfacher Weise entfernen zu können, werden gewöhnlich geeignete Mittel vorgesehen, die das Greifen und Abziehen der Schutzfolie erleichtern können. Eine Möglichkeit besteht beispielsweise darin, einen Bereich zu schaffen, der über den Pflasterrand hinausragt, so dass dieser zum Greifen und Abtrennen der Schutzfolie benutzt werden kann. Hierbei wird jedoch die Schutzfolie als Ganzes von dem Pflaster abgezogen, so dass beim Applizieren das nunmehr nicht mehr von der Schutzfolie bedeckte Pflaster sowohl an seiner Oberseite als auch an seiner Unterseite gegriffen werden muss und dadurch die Finger mit dem wirkstoffhaltigen Reservoir und damit dem Wirkstoff in Kontakt kommen können. Eine solche Kontamination mit dem Wirkstoff sollte natürlich insbesondere bei hochpotenten Wirkstoffen, die nicht unkontrolliert in die Umgebung gelangen sollen, vermieden werden.

Um dieser Kontaminationsgefahr vorzubeugen, werden bei anderen im Stand der Technik bekannten Ausführungsformen die Schutzfolien geteilt ausgeführt. Eine solche geteilte Ausführung der Schutzfolie bringt zwei Vorteile mit sich, zum Einen kann ein Teil der Schutzfolie durch einfaches Verbiegen des Systems abgehoben und gegriffen werden, zum Anderen ist es möglich, zunächst eine Hälfte des Systems auf die Haut aufzukleben und anschließend durch Abziehen der zweiten Hälfte der Schutzfolie die zweite Hälfte des Pflasters aufzubringen, ohne dass beim Applizieren die Finger das wirkstoffhaltige Reservoir berühren müssen.

Für ein einfaches Applizieren wäre es besonders wünschenswert, wenn die beiden Hälften der Schutzfolie etwa gleich groß ausgeführt werden könnten, damit nach dem Entfernen einer der Schutzfolien das Pflaster mit einer relativ großen Fläche auf die Haut aufgeklebt werden kann und sich beim anschließenden Entfernen der zweiten Schutzfolie nicht mehr von der Haut löst. In der Praxis hat sich jedoch gezeigt, dass bei einigen transdermalen therapeutischen Systemen durch den in dieser Ausführungsform in der Mitte des Pflasters befindlichen Spalt bereits bei der Aufbewahrung Wirkstoff aus dem System austreten kann. Zur Lösung dieses Problems wurde im Stand der Technik vorgeschlagen, die Stoßkanten der beiden Schutzfolienhälften (z.B. beim Durchtrennen der Schutzfolie entstehender Schlitz) an den Rand außerhalb der wirkstoffhaltigen Matrix zu verlegen. Hierdurch kann zwar weiterhin vermieden werden, dass beim Aufbringen des Pflasters die Finger mit dem wirkstoffhaltigen Reservoir in Kontakt kommen, das Aufkleben des Pflasters ist jedoch relativ schwierig, da zunächst nach dem Abziehen der ersten kleinen Teilfolie nur ein geringer Teil des Pflasterrands freigelegt wird. Entsprechend gestaltet sich das Aufbringen des zweiten großen Teils des Pflasters auf die Haut schwierig.

In der Offenlegungsschrift DE 199 25 338 A1 wird ein transdermales therapeutisches System mit einer Haftklebeseite beschrieben, die von einer sie zumindest teilweise überragenden Schutzfolie abgedeckt ist. Die Schutzfolie ist aus Gründen der besseren Ablösbarkeit zweiteilig ausgeführt, wobei sich deren beiden Teile im Bereich der Haftklebeseite überlappen und so den Austritt von Haftkleber im kalten Fluss während einer längeren Lagerung verhindern.

Die europäische Patentanmeldung EP 1 967 171 A 1 offenbart ein wirkstoffhaltiges Pflaster zum Aufbringen auf die Haut, dessen haftklebende Oberfläche von einem Schutzfolienpaar bedeckt ist. Wenigstens eine der beiden Schutzfolien ist V-förmig gefaltet und haftet nur mit einem der hierdurch gebildeten Abschnitte auf dem Haftkleber, während der andere Abschnitt als Greifhilfe dient. Die andere Schutzfolie bedeckt den restlichen Teil des Haftklebers und überlappt die V-förmig gefaltete Schutzfolie. Zur Ausbildung der gefalteten Schutzfolie wird eine schlauchförmige Folie auf den abzudeckenden Teil des Pflasters aufgebracht und anschließend am Pflasterrand abgeschnitten. Anschließend wird die zweite so aufgebracht, dass sie die den restlichen Teil der Haftklebeschicht bedeckt und die gefaltete Schutzfolie überlappt.

Die vorliegende Erfindung soll den oben dargelegten Problemen abhelfen und ein transdermales therapeutisches System schaffen, bei dem die Schutzfolie in einfacher Weise abgezogen werden kann, ohne dass die Gefahr besteht, dass die Finger beim Abziehen mit dem wirkstoffhaltigen Reservoir in Kontakt kommen und ohne dass vor der Applikation Wirkstoff aus dem System austreten kann.

Diese Aufgabe wird durch die Gegenstände der unabhängigen Ansprüche gelöst.

Ein erster Aspekt der Erfindung umfasst ein Verfahren zur Herstellung einer zweiteiligen Schutzfolie mit sich überlappenden Kanten für ein transdermales therapeutisches System, bei dem eine als Schutzfolie dienende Folienbahn vor dem Aufbringen auf das wirkstoffhaltige Pflaster zweigeteilt und so geführt wird, dass eine der dabei entstehenden Teilbahnen die andere Teilbahn überlappt.

Ein weiterer Aspekt der Erfindung umfasst ein Verfahren zur Herstellung einer zweiteiligen Schutzfolie mit sich überlappenden Kanten für ein transdermales therapeutisches System mit einem Schritt zum Führen einer als Schutzfolie dienenden Folienbahn über ein Schneidwerkzeug zum Durchtrennen der Folienbahn in Führungsrichtung in eine erste und eine zweite Teilbahn und einem Schritt zum Führen der von der Schnittkante verschiedenen Außenkante der ersten Teilbahn entlang mindestens eines ersten Führungselements, dessen Anordnung die erste Teilbahn von der Führungsrichtung so ablenkt, dass die Schnittkante einer der beiden Teilbahnen unter die andere Teilbahn geführt wird.

Ein weiterer Aspekt der Erfindung umfasst ein Verfahren zur Herstellung eines transdermalen therapeutischen Systems mit einer sich überlappenden Schutzfolie mit einem Schritt zum Aufbringen einer wirkstoffhaltigen Matrix auf eine Trägerfolie und einem Schritt zum Kaschieren einer nach einem wie zuvor angegebenen Verfahren gebildeten zweiteiligen Schutzfolie auf die wirkstoffhaltige Matrix.

Hierdurch wird ein transdermales therapeutisches System mit einer zweigeteilten Schutzfolie geschaffen, von dem jeder Teil in etwa die gleiche Fläche des therapeutischen Pflasters abdecken kann und somit die Voraussetzungen für ein einfaches und sicheres Applizieren des Pflasters gegeben sind, ohne dass der Wirkstoff während der Aufbewahrung durch den Spalt zwischen den beiden Teilen nach außen dringen kann.

Die Herstellung der überlappenden Schutzfolie durch Schneiden einer Folienbahn mit anschließendem Überlappen der beiden Teilbahnen kann einfach und somit kostengünstig in die bestehenden Produktionsabläufe integriert werden. Durch das beschriebene Herstellungsverfahren ist außerdem die Kontamination der Produktionsanlage mit einem Wirkstoff des Pflasters ausgeschlossen.

Ein weiterer Aspekt der Erfindung umfasst eine Vorrichtung zur Herstellung einer zweiteiligen Schutzfolie mit sich überlappenden Kanten für ein transdermales therapeutisches System, das eine Schneidvorrichtung und wenigstens ein Führungselement umfasst, wobei die Schneidvorrichtung zum Durchtrennen einer als Schutzfolie dienenden Folienbahn in Führungsrichtung in eine erste und eine zweite Teilbahn dient und das wenigstens eine Führungselement in Führungsrichtung der Folie nach der Schneidvorrichtung an einer von der Schnittkante verschiedenen Außenkante der ersten Teilbahn so angeordnet ist, dass diese Außenkante abgelenkt und die Schnittkante einer der beiden Teilbahnen unter die andere Teilbahn geführt wird.

Ein weiterer Aspekt umfasst ein transdermales therapeutisches System mit einer Trägerfolie, einer den oder die Wirkstoff(e) enthaltenden Matrix, die auf der Trägerfolie angeordnet ist, und einer aus einer ersten und einer zweiten Teilbahn bestehenden Schutzfolie mit sich überlappenden Kanten, wobei die Schutzfolie eine Dicke besitzt, die im Bereich von 2 bis 1000 µm, insbesondere im Bereich von 10 bis 500 µm, vorzugsweise im Bereich von 10 bis 250 µm und besonders bevorzugt im Bereich von 10 bis 100 µm liegt.

Für ein sicheres Abziehen der Schutzfolie und ein sicheres Aufbringen des transdermalen therapeutischen Systems am Applikationsort ist das TTS vorteilhaft so ausgeführt, dass die erste Teilbahn in Überlappungsrichtung eine Breite b1 aufweist, die höchstens 25 % und vorzugsweise höchstens 10 % von der Breite b2 der zweiten Teilbahn abweicht.

Das Material der Schutzfolie weist vorteilhaft mindestens eine und vorzugsweise alle folgenden Eigenschaften auf: während der Aufbewahrung ausreichende Haftung an der unmittelbar angrenzenden Schicht des Pflasters, leichte Abziehbarkeit während der Verwendung, ausreichende Steifigkeit, um das Übereinanderliegen der Teilbahn im Überlappungsbereich zu gewährleisten, und ausreichende Elastizität für ein sicheres Greifen bei der Applikation.

Vorteilhafte Weiterentwicklungen der Erfindung sind Gegenstand der abhängigen Ansprüche.

Vorteilhaft weist das Verfahren zur Herstellung einer zweiteiligen Schutzfolie einen weiteren Schritt auf zum Führen der von der Schnittkante verschiedenen Außenkante der zweiten Teilbahn entlang mindestens eines zweiten Führungselements, das so angeordnet ist, dass die Schnittkante der zweiten Teilbahn entlang der Führungsrichtung oder in Richtung der ersten Teilbahn geführt wird. Das zweite Führungselement kann dabei bevorzugt bezogen auf die Führungsrichtung der Folienbahn dem ersten Führungselement gegenüber angeordnet sein.

In einer weiteren vorteilhaften Ausführungsform wird die von der Schnittkante verschiedene Außenkante der ersten und/oder der zweiten Teilbahn jeweils über wenigstens ein weiteres Führungselement geführt, das so angeordnet ist, dass die Schnittkante der jeweiligen Teilbahn entlang der Führungsrichtung oder in Richtung der ersten Teilbahn geführt wird.

Vorzugsweise ist das Führungselement als rotationssymmetrischer, drehbar gelagerter Körper ausgebildet. In einer weiteren Ausführungsform umfasst der Schritt zum Führen einer als Schutzfolie dienenden Folienbahn über ein Schneidwerkzeug zweckmäßig das Durchtrennen der Folienbahn in Führungsrichtung in mindestens zwei Paare erster und zweiter Teilfolienbahnen.

Vorzugsweise weisen die ersten Teilfolienbahnen quer zur Führungsrichtung eine Breite b1 auf, die höchstens 25 % und vorzugsweise 10 % von der Breite b2 der zweiten Teilfolienbahnen abweicht.

Eine vorteilhafte Ausführungsform der Vorrichtung zur Herstellung einer zweiteiligen Schutzfolie mit sich überlappenden Kanten für ein transdermales therapeutisches System umfasst vorzugsweise ein zweites Führungselement, das an der von der Schnittkante verschiedenen Außenkante der zweiten Teilbahn so angeordnet ist, dass die Schnittkante der zweiten Teilbahn entlang der Führungsrichtung oder in Richtung der ersten Teilbahn geführt wird. Hierdurch kann die Laufrichtung der zweiten Teilbahn stabilisiert werden. Falls die zweite Teilbahn durch dieses weitere Führungselement in Richtung der ersten Teilbahn geführt wird, kann durch dieses weitere Führungselement die Querbelastung auf die Folienbahn minimiert und einer Faltenbildung vorgebeugt werden, da bei einem vorgegebenen Überlappbereich der Verschiebeweg für jede Teilfolienbahn halbiert ist. Zum einfachen Einstellen der Ablenkwinkel ist das zweite Führungselement bezogen auf die Führungsrichtung der Folienbahn vorzugsweise gegenüber dem ersten Führungselement angeordnet.

Bei einer weiteren vorteilhaften Ausführungsform sind weitere Führungselemente vorgesehen, durch die die von der Schnittkante verschiedene Außenkante von erster bzw. zweiter Teilbahn schrittweise abgelenkt wird, um die Schnittkante der jeweiligen Teilbahn entlang der Führungsrichtung oder in Richtung der ersten Teilbahn zu führen. Diese weiteren Führungselemente sind vorzugsweise paarweise einander gegenüberliegend angeordnet, um die Querbelastung bei beiden Folienteilbahnen gleichmäßig zu gestalten und gering zu halten. Zum Vermindern von Abnutzungen an den Führungselementen sind diese vorteilhaft als rotationssymmetrische und insbesondere vorteilhaft als drehbar gelagerte Körper ausgebildet.

Zur Vermeidung von Faltenbildung bei der Auslenkung der Folienteilbahnen sind die Führungselemente vorzugsweise so angeordnet, dass die Auslenkung der Außenkante der ersten und/oder der zweiten Teilbahn gegenüber der Führungsrichtung jeweils 3,5 Grad nicht überschreitet und insbesondere im Bereich von 0,2 bis 2,78 Grad und vorzugsweise im Bereich von 0,2 bis 0,7 Grad liegt. Auslenkwinkel der Außenkanten und Länge der Versatzstrecke werden vorzugsweise der jeweiligen Pflasterform und Pflastergröße so angepasst, dass der Überlappungsbereich der beiden Folienbahnen wenigstens 10 % oder, um ein sicheres Greifen einer der Folienbahnen zu gewährleisten, mindestens 5 und insbesondere mindestens 7 mm beträgt.

Als Material für die Schutzfolie wird vorzugsweise eine Folienbahn gewählt, die mindestens eine und vorzugsweise alle folgenden Eigenschaften aufweist: während der Aufbewahrung ausreichende Haftung an der unmittelbar angrenzenden Schicht des Pflasters, leichte Abziehbarkeit während der Verwendung, ausreichende Steifigkeit, um das Übereinanderliegen der Teilbahn im Überlappungsbereich zu gewährleisten, und ausreichende Elastizität für ein sicheres Greifen bei der Applikation. Natürlich darf die Schutzfolie für den Wirkstoff nicht durchlässig sein.

Weitere Merkmale der Erfindung ergeben sich aus der folgenden Beschreibung von Ausführungsbeispielen in Verbindung mit den Ansprüchen sowie der Figuren. Die einzelnen Merkmale können bei einer Ausführungsform gemäß der Erfindung je für sich oder zu mehreren verwirklicht sein. Bei der nachfolgenden Erläuterung einiger Ausführungsbeispiele der Erfindung wird auf die beiliegenden Figuren Bezug genommen, von denen
- Fig. 1: eine schematische Darstellung einer Vorrichtung zur Herstellung einer zweiteiligen Schutzfolie mit sich überlappenden Kanten in einer Seitenansicht zeigt,
- Fig. 2: in einer schematischen Darstellung die Vorrichtung von Fig. 1 in einer Draufsicht zeigt,
- Fig. 3: ein Flussdiagramm zeigt, in dem die wesentlichen Schritte des von der Vorrichtung von Fig. 1 und Fig. 2 durchgeführten Verfahrens dargestellt sind, und
- Fig. 4: ein transdermales therapeutisches System mit einer zweiteiligen sich überlappenden Schutzfolie in einer Untenansicht und einer Querschnittsansicht veranschaulicht.

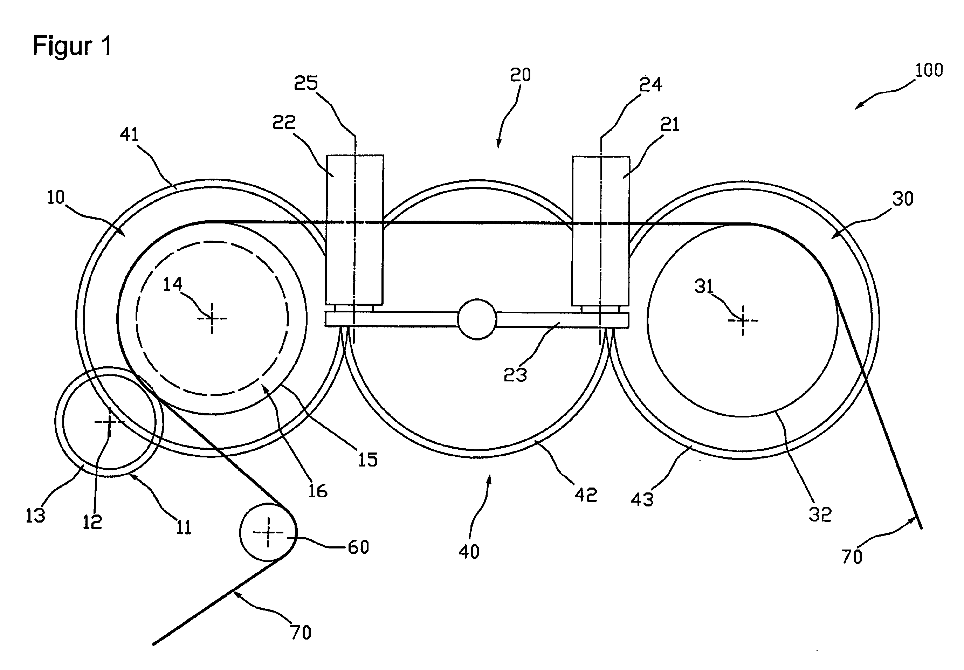

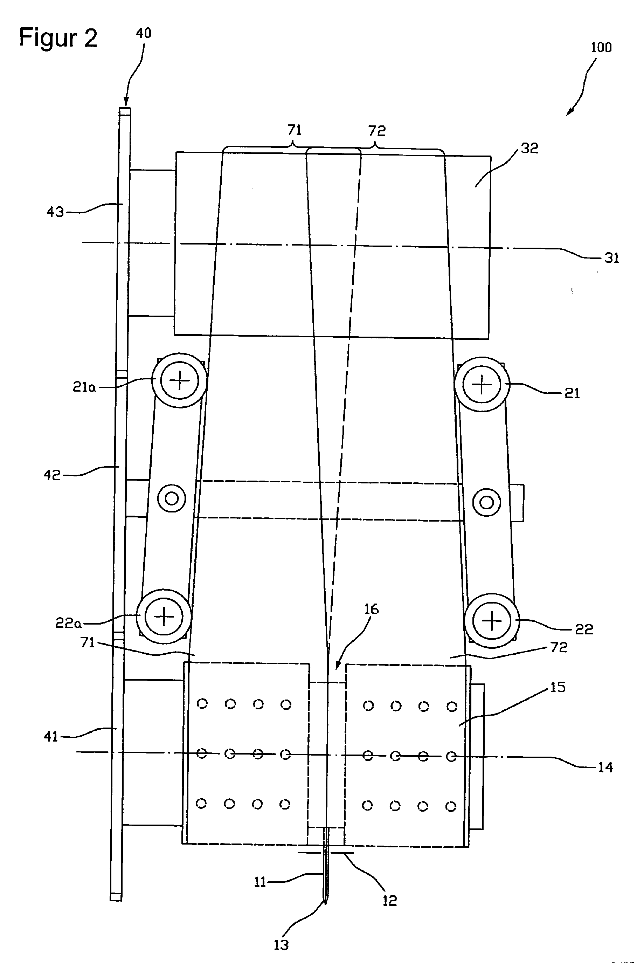

Fig. 1 zeigt eine Vorrichtung zur Herstellung einer zweiteiligen Schutzfolie mit sich überlappenden Kanten für ein transdermales therapeutisches System in einer Seitenansicht. In der Fig. 2 ist diese Vorrichtung in einer Draufsicht veranschaulicht. Die Figuren zeigen nur diejenigen Komponenten, die für das Verständnis der vorliegenden Erfindung wesentlich sind. Auf die Darstellung weiterer Komponenten, die für die Ausführung der Vorrichtung erforderlich, für die Beschreibung der Erfindung jedoch nicht wesentlich sind, wurde im Hinblick auf eine übersichtliche Darstellung verzichtet. Dennoch sind diese Komponenten als vorhanden anzusehen und dem Fachmann bekannt.

Die Vorrichtung 100 umfasst eine Schneidvorrichtung 10, eine Kantenführungseinrichtung 20 und eine Aufnahmeeinrichtung 30. Eine als Schutzfolie dienende Folienbahn 70 wird der Schneidvorrichtung 10 zum Beispiel über eine Umlenkrolle 60 wie im vorgestellten Fall zugeführt. Die Schneidvorrichtung 10 weist eine Transportwalze 15 auf, über deren Mantelfläche die Folienbahn 70 mit einem bestimmten Umschlingungswinkel geführt wird. Während des Transports der Folienbahn dreht sich die Transportwalze 15 um die Achse 14, so dass es zu keiner Relativverschiebung der Folienbahn 70 gegenüber der Mantelfläche der Transportwalze 15 kommen kann. Die Transportwalze 15 weist eine umlaufende ringförmige Ausnehmung in Form einer Rille 16 auf, in die ein Schneidwerkzeug 11 eingreift. Hierdurch wird die über die Transportwalze 15 geführte Folienbahn 70 mit einem Längsschnitt versehen, der sich in Förderrichtung der Folienbahn 70 erstreckt.

Das Schneidwerkzeug kann in einer einfachen mit einer Schneide versehenen stehenden Klinge bestehen. Zur Ausbildung eines glatten Schnittrands über die gesamte Länge der Folienbahn wird jedoch vorzugsweise eine rotierende Klinge mit einer wie in Fig. 1 dargestellten rotationssymmetrischen Geometrie eingesetzt. Die Rotation der Klinge 13 um die Achse 12 der Schneidvorrichtung 11 wird über einen in der Figur nicht dargestellten Antrieb bewirkt.

Die in ihrer Längsrichtung in zwei Teilbahnen geschnittene Folienbahn 70 wird anschließend durch die Kantenführungseinrichtung 20 zu einer Aufnahmeeinrichtung 30 geführt. Die Rotationsgeschwindigkeiten von Transportwalze 15 und Aufnahmewalze 32 der Aufnahmeeinrichtung 30 sind dabei so aufeinander abgestimmt, dass die Folienbahn zwischen den beiden Walzen gespannt ist, ohne auf einer der beiden Walzen zu rutschen.

Die Kantenführungseinrichtung 20 weist wenigstens ein Führungselement 21 oder 22 auf, das gegen die nicht von der Schnittkante gebildete Außenkante einer der beiden Teilbahnen seitlich zur Führungsrichtung drückt, wodurch diese Teilbahn zur anderen Teilbahn hin verschoben wird. Die Teilbahn kann dabei unter oder auch über die andere Teilbahn geführt werden. Welche Teilbahn die andere überlappt, wird beim Einrichten der Folienbahn in die Vorrichtung 100 festgelegt, indem die entsprechende Teilbahn unterhalb oder oberhalb der anderen Teilbahn angeordnet wird. Nach dem anfänglichen Einrichten bleibt die relative Lage der Teilbahnen zueinander durch die Führung der Folienbahn 70 und die Führungselemente 21 bzw. 22 erhalten.

Da die als Schutzfolie dienende Folienbahn 70 mit Dicken üblicherweise im Bereich von 2 bis 1.000 µm dünn ist, können sich an der Berührungsstelle zwischen Folienbahn 70 und Führungselement 21 bzw. 22 sehr hohe lokale Drücke ausbilden, die beim Transport der Folienbahn 70 zu Schleifspuren an der Führungseinrichtung 21 bzw. 22 führen können, wodurch die Überlappung der beiden Folienteilbahnen mit der Zeit geringer wird. Um dies zu vermeiden, ist das Führungselement 21 bzw. 22 vorzugsweise um eine Achse 24 bzw. 25 drehbar ausgeführt, wobei zum Erreichen einer konstanten Überlappung der beiden Teilfolienbahnen das Führungselement 21 bzw. 22 rotationssymmetrisch ausgebildet ist. Das oder die Führungselement(e) der Kantenführungseinrichtung sind vorzugsweise auf einer Halterung montiert, wobei bei Verwendung mehrerer Führungselemente an einer Außenkante einer Folienbahn diese Führungselemente vorzugsweise auf einem Halteelement 23 montiert sind, das eine gleichmäßig geneigte Justierung der Führungselemente gestattet.

Die Aufsichtsdarstellung der Vorrichtung in Fig. 2 veranschaulicht die Teilung der Folienbahn 70 in zwei Teilbahnen 71 und 72, die über die Kantenführungseinrichtung 20 zueinander so verschoben werden, dass ein Teil einer der beiden Teilbahnen über einen Teil der anderen Teilbahn zu liegen kommt und in dieser Überlagerung von der Aufnahmewalze 32, die sich um die Achse 31 dreht, aufgenommen wird. Im vorgestellten Fall wird die Folienbahn 70 von unten an die Transportwalze 15 herangeführt, und liegt an der Mantelfläche der Transportwalze an, bevor die Klinge 13 des Schneidwerkzeugs 11 die Folienbahn durchtrennt. Die Klinge 13 greift in die in Umfangsrichtung der Transportwalze 15 verlaufende Rille 16 ein und kann die Folienbahn 70 somit vollständig durchtrennen. Die Einstellung der Schnitttiefe in der Klinge 13 ist hierbei unkritisch, da die nach dem Schnitt entstandenen Folienteilbahnen 71 und 72 nicht auf Stoß aneinanderliegend weitergeführt werden, sondern mit Hilfe der Kantenführungseinrichtung sich teilweise überlappend übereinander geführt werden.

Im vorgestellten Ausführungsbeispiel weist die Kantenführungseinrichtung vier Führungselemente 21, 21A, 22 und 22A auf, die sich jeweils mit Bezug auf die Führungs- bzw. Längsrichtung der Folienbahn paarweise gegenüber liegen. Mit anderen Worten werden beide Außenkanten, d. h. die nicht von der Schnittkante gebildeten Kanten der Teilfolienbahnen 71 bzw. 72 entlang der Führungselemente geführt. Dies ist nicht unbedingt erforderlich, ermöglicht jedoch eine sehr genaue und stabile oszillationsfreie Ablenkung der Teilfolienbahnen aus der Führungsrichtung. Gemäß einer Ausführungsform dienen die Führungselemente an der Außenkante einer der beiden Teilbahnen zur Ablenkung dieser Teilbahnen, beispielsweise die Führungselemente 21 und 22, während die Führungselemente an der anderen Teilbahn verhindern sollen, dass bei der Überlappung eventuell auf diese Teilbahn übertragene Schubkräfte ebenfalls zu einer Auslenkung dieser Teilbahn führen, die somit die Überlappung verringern würde. In einer anderen Ausführungsform, die in Fig. 2 gezeigt ist, werden jedoch beide Teilbahnen unabhängig voneinander über die Führungselemente an ihren Außenkanten zur Folienbahnmitte verschoben bzw. ausgelenkt, so dass die resultierende Überlappung als Summe der Auslenkung beider Folienteilbahnen bewirkt wird.

Bei Folienbahnen geringer Steifigkeit erfolgt die Ablenkung der Teilbahnen hin zur Folienbahnmitte in der Regel gestuft, d. h. so, dass der Ablenkwinkel stufenweise von einem Führungselement zum nächsten erhöht wird. In diesem Falle sind die von der Außenkante einer Folienbahn berührten Punkte der Führungselemente nicht entlang einer geraden, sondern entlang einer gekrümmten Kurve angeordnet. Hierdurch können die Querkräfte auf die Folienteilbahnen minimiert und somit einer Faltenbildung vorgebeugt werden.

Bei starren Folien können die beiden Führungselemente 21 und 22 bzw. 21A und 22A entlang einer parallel zur Führungsrichtung verlaufenden Geraden angeordnet werden, wobei der Abstand zwischen den Führungselementen 21 und 21A sowie 22 und 22A nicht nur konstant, sondern auch kleiner als die Breite der Folienbahn 70 ist, so dass bereits das erste der beiden Führungselementpaare die Überlappung der beiden Teilfolienbahnen bewirkt, während das zweite Führungselementpaar 21 und 21A diese Überlappung nur aufrecht erhält und somit keine Querspannungen zwischen diesem letzten Führungspaar und der Aufnahmerolle entstehen können. Selbstverständlich kann diese Ausführungsform mit der zuvor beschriebenen oder in Fig. 2 dargestellten kombiniert werden, so dass die Kantenführungseinrichtung wenigstens zwei Führungselementpaare besitzt, die in Führungsrichtung der Folienbahn als letzte angeordnet sind und die beide den gleichen durch die Überlappung bestimmten Abstand aufweisen.

Bei der in Fig. 2 veranschaulichten Ausführungsform sind die Führungselemente auf jeder der beiden Außenkanten der Teilfolienbahnen jeweils auf einer geneigt zur Führungsrichtung verlaufenden Geraden angeordnet und bewirken somit eine stabile allmähliche Übereinanderführung der beiden Teilfolienbahnen 71 und 72.

Durch die vorgestellten Kantenführungseinrichtungen werden die mit Hilfe des Schneidwerkzeugs 11 erzeugten Folienteilbahnen 71 und 72 übereinander geführt, sobald die Folienbahn nicht mehr auf der Mantelfläche der Transportwalze 15 aufliegt. Bei dickeren und vor allem steiferen Folienbahnen 70 besteht jedoch eine gewisse Gefahr, dass die von den Führungselementen auf die Folienteilbahnen 71 und 72 ausgeübten Querkräfte auch auf den Teil der Folienbahn zurück wirken, der auf der Mantelfläche der Transportwalze 15 aufliegt, wodurch es zu einem Aufstellen der Schnittkanten der Folienteilbahnen mit der Folge einer unsauberen Schnittführung an der Stelle der Klinge 13 kommen kann. Um diesem Effekt vorzubeugen, ist die Transportwalze 15 vorzugsweise mit Bohrungen versehen, die in der Fig. 2 als Reihen kleiner Kreise angedeutet sind und die Oberfläche der Transportwalze mit einer Saugeinrichtung verbinden. Durch den hierbei entstehenden Unterdruck werden die Folienbahnen auf der Mantelfläche der Transportwalze 15 fixiert, so dass sie sich auf der Mantelfläche nicht in Richtung ihrer Schnittkanten verschieben können.

Der Antrieb von Transportwalze und Aufnahmewalze erfolgt bevorzugt aktiv und synchronisiert. Zur Synchronisation der Drehgeschwindigkeiten der beiden Walzen sind diese wie in Fig. 2 erkennbar vorzugsweise über ein Getriebe 40 miteinander verbunden. Das Getriebe 40 weist beispielsweise ein Ritzel 43 auf, dessen Umdrehung über das Zahnrad 42 auf das fest mit der Transportwalze 15 verbundene Zahnrad 41 übertragen wird. Die Übersetzung der Drehgeschwindigkeit erfolgt dabei so, dass die Bahngeschwindigkeiten auf den Mantelflächen der beiden Walzen, d. h. der Aufnahmewalze 32 und der Transportwalze 15 identisch sind, wodurch Zuführung und Abführung der Folienteilbahn 71 und 72 zur bzw. von der Kantenführungseinrichtung mit gleichen Geschwindigkeiten erfolgt und somit eine Dehnung oder ein Durchhängen der Folienbahn in der Kantenführungseinrichtung vermieden wird. Weisen Transportwalze 15 und Aufnahmewalze 32 wie im dargestellten Fall gleiche Durchmesser auf, so ist die Übersetzung 1:1, und die Durchmesser von Ritzel 43 und Zahnrad 41 entsprechen sich. Die Größe des übertragenden Zahnrads 42 wird wesentlich von den Größen der beiden anderen Zahnräder und der Länge der Kantenführungseinrichtung 20 bestimmt. Bei Verwendung sehr geringer Ablenkwinkel oder großer Überlappungen kann die Länge der Kantenführungseinrichtung sehr große Werte einnehmen, so dass das Zwischengetriebe 42 vorzugsweise über eine ungerade Anzahl an Zwischenzahnrädern realisiert wird.

Nach einer bevorzugten Ausführungsform dient die Aufnahmewalze dazu, das Laminat aus Wirkstoffreservoir und Rückschicht auf die sich überlappende zweigeteilte Schutzfolie zu kaschieren. Selbstverständlich kann die zweigeteilte sich überlappende Schutzfolie für eine spätere Verwendung im Anschluss an die Aufnahmewalze auf eine Vorratsrolle aufgewickelt werden.

Bei der in Fig. 2 dargestellten Ausführungsform wird die Folienbahn 70 in etwa in ihrer Mitte geschnitten, so dass zwei gleich breite Folienbahnen 71 und 72 entstehen. Die relativen Breiten der Folienteilbahnen 71 und 72 bestimmen sich jedoch nach dem jeweiligen Anwendungsfall und können natürlich stark voneinander abweichen. Vorzugsweise weichen die Breiten der beiden Teilbahnen jedoch höchstens 25 % und vorzugsweise höchstens 10 % voneinander ab, wodurch nach dem Abziehen der ersten Teilbahn eine genügend große Fläche zum sicheren Aufbringen des transdermalen therapeutischen Systems auf die Haut zur Verfügung steht und auch der zweite Teil der Schutzfolie leicht abgezogen werden kann. Der Überlappungsbereich wird vorzugsweise so festgelegt, dass die überstehende Schutzfolie leicht mit den Fingern gegriffen und abgezogen werden kann, ohne den wirkstoffhaltigen Teil des transdermalen therapeutischen Systems zu berühren. Bei einer Überlappung von mindestens 5 mm ist die Wahrscheinlichkeit eines ungewollten Berührens einer wirkstoffhaltigen Oberfläche des TTS bereits verschwindend gering. Vorzugsweise wird eine Überlappung von mindestens 7 mm eingestellt, die es auch Personen mit geringer Feinmotorik ermöglicht, die Folie gut greifen zu können.

Damit die Folienbahnen der Schutzfolie vollständig auf der Klebefläche des transdermalen Systems aufliegen, dürfen die Folienbahnen der Schutzfolie keine Falten aufweisen. Um dies zu erreichen, ist die mit den Führungselementen der Kantenführungseinrichtung 20 bewirkte Auslenkung der Außenkanten der Folienteilbahnen 71 und 72 gegenüber der Führungsrichtung vorzugsweise auf jeweils maximal 3,5 Grad beschränkt, wobei ein Bereich der Auslenkung von 0,2 bis 2,78 Grad bevorzugt wird und insbesondere eine Auslenkung im Bereich von 0,2 bis 0,7 Grad besonders bevorzugt wird.

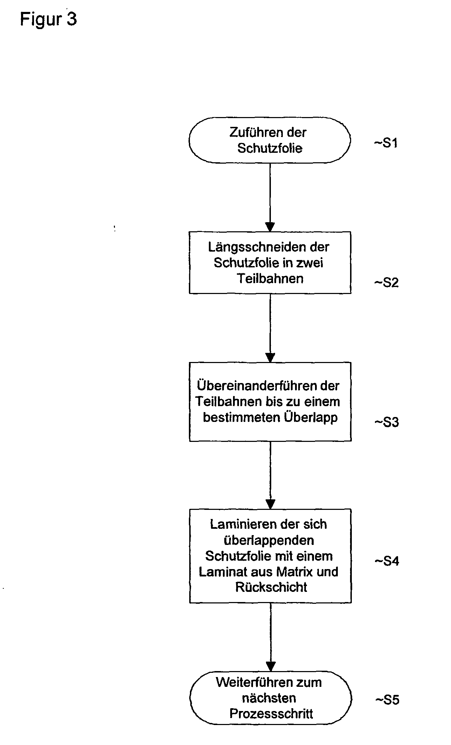

In dem Flussdiagramm der Fig. 3 sind die wesentlichen Verfahrensschritte aufgeführt, die von der mit Bezug auf die Fig. 1 und 2 beschriebenen Vorrichtung ausgeführt werden. Das Verfahren beginnt in Schritt S1 mit dem Zuführen der Schutzfolienbahn 70 zur Schneidvorrichtung 10. Im nachfolgenden Schritt S2 schneidet die Schneidvorrichtung 10 die Folienbahn 70 in zwei Teilfolienbahnen 71 und 72, die in Schritt S3 mit Hilfe der Kantenführungseinrichtung 20 so aufeinander zugeführt werden, dass im Bereich der Schnittkante eine der beiden Folienteilbahnen teilweise über die andere Folienteilbahn geführt wird. Im anschließenden fakultativen Schritt S4 wird ein Laminat aus wirkstoffhaltiger Matrix und Trägerfolie auf die sich überlappenden Teilfolienbahnen auflaminiert, bevor das Verfahren in Schritt S5 endet bzw. an ein nachfolgendes Verpackungsverfahren anschließt.

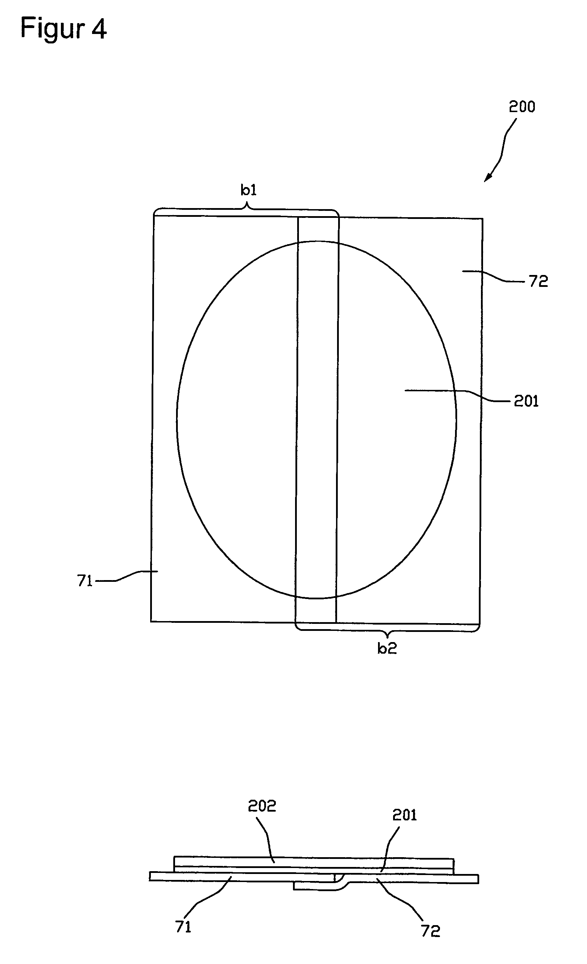

Ein mit Hilfe des beschriebenen Verfahrens und der beschriebenen Vorrichtung hergestelltes transdermales therapeutisches System ist in Fig. 4 veranschaulicht. Das hier gezeigte System 200 besteht aus einer Rückfolie 202, einer den Wirkstoff enthaltenden Matrix 201 auf der Basis eines selbsthaftenden Klebstoffes sowie der aus den beiden Folienbahnen 71 und 72 bestehenden, sich überlappenden Schutzfolie. Die wirkstoffhaltige Matrix weist ein Flächengewicht auf, das im Bereich von 40 bis 400 g/m² liegt. Das Flächengewicht liegt bevorzugt im Bereich von 50 bis 350 g/m² und insbesondere im Bereich von 80 bis 250 g/m².

In dem gezeigten Beispiel handelt es sich um eine selbsthaftende Matrix aus einem Polyacrylatklebstoff, die den Wirkstoff Buprenorphin enthält. Selbstverständlich ist die sich überlappende Schutzfolie auch für weitere transdermale therapeutische Systeme geeignet, Beispiele hierfür sind etwa Matrixsysteme, die mit einer Klebeschicht versehen sind, Reservoirsysteme, deren Wirkstoffabgabe durch eine Membran reguliert wird, Mehrschichtsysteme, deren Schichten den Wirkstoff in unterschiedlichen Konzentrationen enthalten und dergleichen. Ist die den Wirkstoff enthaltende Schicht nicht selbsthaftend ausgeführt, kann hautseitig eine Klebeschicht vorgesehen werden, das System kann aber zusätzlich auch ein so genanntes Obertape aufweisen, mit dem das gesamte System klebend auf der Haut befestigt werden kann. Das Material und die Materialeigenschaften der Rückschicht werden in üblicher Weise in Abhängigkeit von dem jeweiligen System und seiner Größe gewählt, vorzugsweise ist die Rückschicht für den Wirkstoff undurchlässig.

Unter dem Begriff 'wirkstoffhaltige Matrix' sind, wie er gemäß der vorliegenden Erfindung verwendet wird, nicht nur die eigentlichen, den Wirkstoff in einer Polymermatrix enthaltenden Matrixsysteme zu verstehen, sondern es sollen auch die Reservoirsysteme mit umfasst werden, bei denen ferner eine die Wirkstofffreisetzung regulierende Membran vorhanden ist, wobei sich die Erfindung insbesondere auf die eigentlichen Matrixsysteme bezieht.

Grundsätzlich können alle dem Fachmann bekannten transdermalen therapeutischen Systeme mit der wie oben beschriebenen zweiteiligen Schutzfolie ausgestattet werden. Besonders vorteilhaft ist die Schutzfolie für Systeme, die einen Wirkstoff enthalten, der keinesfalls unkontrolliert in die Umgebung gelangen soll. Beispiele hierfür sind etwa die als Analgetika dienenden Opioide Fentanyl, Buprenorphin, Remifentanil, Sulfentanil, die dem Betäubungsmittelgesetz unterliegen und besonders heikel zu handhaben sind.

Nach einer weiteren bevorzugten Ausführungsform wird die wie oben beschriebene zweiteilige, sich überlappende Schutzfolie bei Systemen verwendet, deren Matrix einen hohen kalten Fluss aufweist, so dass bei diesen Systemen der Austritt von Matrix und dem darin enthaltenen Wirkstoff und somit beispielsweise auch das Verkleben der das Pflaster enthaltenden Umverpackung wirksam vermieden werden kann.

Das in Fig. 4 gezeigte transdermale therapeutische System weist eine rechteckige Form auf, wobei die Kantenlängen 10 cm bzw. 8 cm betragen. Die Breite der ersten Teilbahn b 1 beträgt 4,5 cm und entspricht der Breite b2 der zweiten Teilbahn.

Das Material der Schutzfolie wird so gewählt, dass es während der Aufbewahrung sicher auf dem Laminat aus wirkstoffhaltigem Reservoir und Rückschicht haftet, jedoch bei der Anwendung leicht abgezogen werden kann. Vorzugsweise grenzt die Schutzfolie unmittelbar an eine Klebeschicht des transdermalen therapeutischen Systems an, besonders bevorzugt an eine selbsthaftende, den Wirkstoff enthaltende Polymermatrix. Geeignete Materialien für die Schutzfolie sind beispielsweise Polyester, Polypropylen, Polyvinylchlorid, Aluminium und Papier, wobei zumindest eine Seite der Schutzfolie eine Siliconbeschichtung, Polyethylenbeschichtung, Fluorsiliconbeschichtung oder Polytetrafluorethylenbeschichtung aufweist. Die Dicke der Schutzfolie ist an die Steifigkeit des Materials der Schutzfolie angepasst. Sie muss selbsttragend sein, eine ausreichende Steifigkeit besitzen, damit das Übereinanderliegen der beiden Teilbahnen im Überlappungsbereich gewährleistet ist, und die Elastizität muss ausreichend sein, um ein sicheres Greifen zu gewährleisten. Hierfür wird die Dicke der Schutzfolie im Bereich von 2 bis 1.000 µm, insbesondere im Bereich von 10 bis 500 µm und vorzugsweise im Bereich von 15 bis 100 µm gewählt.

Durch die Erfindung ist es möglich, ein transdermales therapeutisches System zu schaffen, dass ein einfaches und sicheres Aufbringen des Systems auf die Haut ohne Kontamination der das Pflaster applizierenden Person oder der Umverpackung des Pflasters ermöglicht.

## Patentansprüche

1. Verfahren zur Herstellung einer zweiteiligen Schutzfolie mit sich überlappenden Kanten für ein transdermales therapeutisches System mit den folgenden Schritten:
- Führen einer als Schutzfolie dienenden Folienbahn (70) über ein Schneidwerkzeug (11) zum Durchtrennen der Folienbahn (70) in Führungsrichtung in eine erste Teilbahn (71) und eine zweite Teilbahn (72),
- Führen der von der Schnittkante verschiedenen Außenkante der ersten Teilbahn (71) entlang mindestens eines ersten Führungselements (22a), dessen Anordnung die erste Teilbahn (71) von der Führungsrichtung so ablenkt, dass die Schnittkante einer der beiden Teilbahnen unter die andere Teilbahn geführt wird.

2. Verfahren nach Anspruch 1 mit einem weiteren Schritt zum Führen der von der Schnittkante verschiedenen Außenkante der zweiten Teilbahn (72) entlang mindestens eines zweiten Führungselements (22), das so angeordnet ist, dass die Schnittkante der zweiten Teilbahn (72) entlang der Führungsrichtung oder in Richtung der ersten Teilbahn geführt wird.

3. Verfahren nach Anspruch 2, wobei das zweite Führungselement (22) bezogen auf die Führungsrichtung der Folienbahn dem ersten Führungselement (22a) gegenüber angeordnet ist.

4. Verfahren nach einem der vorhergehenden Ansprüche, wobei die von der Schnittkante verschiedene Außenkante der ersten und/oder der zweiten Teilbahn (71, 72) jeweils über wenigstens ein weiteres Führungselement (21a, 21) geführt wird, das so angeordnet ist, dass die Schnittkante der jeweiligen Teilbahn entlang der Führungsrichtung oder in Richtung der ersten Teilbahn geführt wird.

5. Verfahren nach einem der vorhergehenden Ansprüche, wobei das Führungselement (21, 22, 21a, 22a) als rotationssymmetrischer, drehbar gelagerter Körper ausgebildet ist.

6. Verfahren nach einem der vorhergehenden Ansprüche, wobei der Schritt zum Führen einer als Schutzfolie dienenden Folienbahn über ein Schneidwerkzeug das Durchtrennen der Folienbahn in Führungsrichtung in mindestens zwei Paare erster und zweiter Teilfolienbahnen umfasst.

7. Verfahren nach einem der vorhergehenden Ansprüche, wobei die ersten Teilfolienbahnen quer zur Führungsrichtung eine Breite b1 aufweisen, die höchstens 25 % und vorzugsweise 10 % von der Breite b2 der zweiten Teilfolienbahnen abweicht.

8. Verfahren zur Herstellung eines transdermalen therapeutischen Systems (200) mit einer sich überlappenden Schutzfolie mit den folgenden Schritten:
- Aufbringen einer wirkstoffhaltigen Matrix (201) auf eine Trägerfolie (202),
- Kaschieren einer gemäß einem Verfahren nach einem der vorhergehenden Ansprüche gebildeten zweiteiligen Schutzfolie auf die wirkstoffhaltige Matrix (201).

9. Vorrichtung zur Herstellung einer zweiteiligen Schutzfolie mit sich überlappenden Kanten für ein transdermales therapeutisches System, die umfasst:
- eine Scheidvorrichtung (11) zum Durchtrennen einer als Schutzfolie dienenden Folienbahn in Führungsrichtung in eine erste Teilbahn (71) und eine zweite Teilbahn (72),
- wenigstens ein Führungselement (22a), das in Führungsrichtung der Folie nach der Schneidvorrichtung (11) an einer von der Schnittkante verschiedenen Außenkante der ersten Teilbahn (71) so angeordnet ist, dass diese Außenkante abgelenkt und die Schnittkante einer der beiden Teilbahnen (71, 72) unter die andere Teilbahn geführt wird.

10. Vorrichtung nach Anspruch 9, die ein zweites Führungselement (22) umfasst, das an der von der Schnittkante verschiedenen Außenkante der zweiten Teilbahn (72) so angeordnet ist, dass die Schnittkante der zweiten Teilbahn (72) entlang der Führungsrichtung oder in Richtung der ersten Teilbahn geführt wird.

11. Vorrichtung nach Anspruch 9 oder 10, wobei das zweite Führungselement (22) bezogen auf die Führungsrichtung der Folienbahn dem ersten Führungselement (22a) gegenüber angeordnet ist.

12. Vorrichtung nach einem der Ansprüche 9 bis 11, die mindestens ein weiteres Führungselement (21a, 21) umfasst, durch das die von der Schnittkante verschiedene Außenkante der ersten und/oder der zweiten Teilbahn und dadurch die Schnittkante der jeweiligen Teilbahn entlang der Führungsrichtung oder in Richtung der anderen Teilbahn geführt wird.

13. Vorrichtung nach einem der Ansprüche 9 bis 12, wobei das Führungselement (21, 22, 21a, 22a) als rotationssymmetrischer, drehbar gelagerter Körper ausgebildet ist.

14. Vorrichtung nach einem der Ansprüche 9 bis 13, wobei die Auslenkung der Außenkante der ersten und/oder der zweiten Teilbahn (71, 72) gegenüber der Führungsrichtung jeweils 3,5 Grad nicht überschreitet und insbesondere im Bereich von 0,2 bis 2,78 Grad und vorzugsweise im Bereich von 0,2 bis 0,7 Grad liegt.

## Claims

1. A method for the production of a two-part protection film with overlapping edges for a transdermal therapeutic system, which comprises the following steps:
- guiding a film web (70) serving as a protection film over a cutting tool (11) for cutting the film web (70) in the guiding direction into a first partial web (71) and a second partial web (72),
- guiding the outer edge of the first partial web (71), which is different from the cutting edge, along at least a first guiding element (22a), the arrangement of which makes the first partial web (71) deviate from the guiding direction in such a way that the cutting edge of one of the two partial webs is guided below the other partial web.

2. The method according to claim 1 comprising a further step for guiding the outer edge of the second partial web (72), which is different from the cutting edge, along at least a second guiding element (22) which is arranged in such a way that the cutting edge of the second partial web (72) is moved along the guiding direction or in the direction of the first partial web.

3. The method according to claim 2, wherein the second guiding element (22) is arranged opposite to the first guiding element (22a) with respect to the guiding direction.

4. The method according to one of the preceding claims, wherein the outer edge of the first and/or the second partial web (71, 72), which is different from the cutting edge, is respectively guided over at least one further guiding element (21a, 21) which is arranged in such a way that the cutting edge of the respective partial web is guided along the guiding direction or in the direction of the first partial web.

5. The method according to one of the preceding claims, wherein the guiding element (21, 22, 21a, 22a) is formed as a rotationally symmetric, rotatably supported body.

6. The method according to one of the preceding claims, wherein the step for guiding a film web serving as a protection film over a cutting tool comprises the cutting of the film web in the guiding direction into at least two pairs of first and second partial film webs.

7. The method according to one of the preceding claims, wherein the first partial film webs have a width b 1 transversely to the guiding direction, which deviates by maximally 25 % and preferably 10 % from the width b2 of the second partial film webs.

8. A method for the production of a transdermal therapeutic system (200) with an overlapping protection film, which comprises the following steps:
- applying a matrix (201) containing an active substance to a support film (202),
- masking a two-part protection film formed compliant with a method according to one of the preceding claims on the matrix (201) containing the active substance.

9. Device for the production of a two-part protection film with overlapping edges for a transdermal therapeutic system, which comprises:
- a cutting device (11) for cutting a film web serving as a protection film in the guiding direction into a first partial web (71) and a second partial web (72),
- at least one guiding element (22a) which is arranged in the guiding direction of the film downstream of the cutting device (11) at an outer edge of the first partial web (71), which is different from the cutting edge, in such a way that this outer edge is diverted and that the cutting edge of one of the two partial webs (71, 72) is guided below the other partial web.

10. The device according to claim 9, which comprises a second guiding element (22) which is arranged at the outer edge of the second partial web (72), which is different from the cutting edge, in such a way that the cutting edge of the second partial web (72) is guided along the guiding direction or in the direction of the first partial web.

11. The device according to claim 9 or 10, wherein the second guiding element (22) is arranged opposite to the first guiding element (22a) with respect to the guiding direction of the film web.

12. The device according to one of the claims 9 to 11, which comprises at least one further guiding element (21a, 21) by which the outer edge of the first and/or the second partial web, which is different from the cutting edge, and thereby the cutting edge of the respective partial web is guided along the guiding direction or in the direction of the other partial web.

13. The device according to one of the claims 9 to 12, wherein the guiding element (21, 22, 21a, 22a) is formed as a rotationally symmetric, rotationally supported body.

14. The device according to one of the claims 9 to 13, wherein the displacement of the outer edge of the first and/or the second partial web (71, 72) relative to the guiding direction does not exceed 3.5 degrees and, in particular, lies within the range of 0.2 to 2.78 degrees and, preferably, in the range of 0.2 to 0.7 degrees, respectively.

## Revendications

1. Procédé de fabrication d'une feuille de protection en deux parties avec des arêtes se chevauchant pour un système thérapeutique transdermique, comprenant les étapes suivantes :
- guidage d'une bande de feuille (70) servant de feuille de protection au-dessus d'un outil de coupe (11) pour séparer la bande de feuille (70) dans la direction du guidage en une première bande partielle (71) et en une deuxième bande partielle (72),
- guidage de l'arête externe de la première bande partielle (71), différente de l'arête de coupe, le long d'au moins un premier élément de guidage (22a), dont la disposition fait dévier la première bande partielle (71) de la direction du guidage de telle sorte que l'arête de coupe d'une des deux bandes partielles soit guidée en dessous de l'autre bande partielle.

2. Procédé selon la revendication 1 comprenant une autre étape pour le guidage de l'arête externe de la deuxième bande partielle (72), différente de l'arête de coupe, le long d'au moins un deuxième élément de guidage (22), qui est disposé de telle sorte que l'arête de coupe de la deuxième bande partielle (72) est guidée le long de la direction de guidage ou dans la direction de la première bande partielle.

3. Procédé selon la revendication 2, dans lequel le deuxième élément de guidage (22) est disposé par rapport à la direction de guidage de la bande de feuille en face du premier élément de guidage (22a).

4. Procédé selon l'une des revendications précédentes, dans lequel l'arête externe de la première et/ou de la deuxième bande partielle (71, 72), différente de l'arête de coupe, est guidée respectivement au-dessus d'au moins un autre élément de guidage (21a, 21) qui est disposé de telle sorte que l'arête de coupe de chaque bande partielle est guidée le long de la direction de guidage ou dans la direction de la première bande partielle.

5. Procédé selon l'une des revendications précédentes, dans lequel l'élément de guidage (21, 22, 21a, 22a) prend la forme d'un corps rotatif à symétrie de rotation.

6. Procédé selon l'une des revendications précédentes, dans lequel l'étape de guidage d'une bande de feuille servant de feuille de protection au-dessus d'un outil de coupe comprend la séparation de la bande de feuille dans la direction du guidage en au moins deux paires de premières et deuxièmes bandes de feuilles partielles.

7. Procédé selon l'une des revendications précédentes, dans lequel les premières bandes de feuille partielles présentent, dans le sens transversal à la direction du guidage, une largeur b1 qui diverge au maximum de 25 % et de préférence de 10 % de la largeur b2 des deuxièmes bandes de feuille partielles.

8. Procédé de fabrication d'un système thérapeutique transdermique (200) avec une feuille de protection se chevauchant, comprenant les étapes suivantes :
- dépôt d'une matrice contenant un principe actif (201) sur une feuille support (202),
- contre-collage d'une feuille de protection en deux parties formée selon un procédé d'après l'une des revendications précédentes sur la matrice contenant un principe actif (201).

9. Dispositif de fabrication d'une feuille de protection en deux parties avec des arêtes se chevauchant pour un système thérapeutique transdermique, comprenant :
- un dispositif de coupe (11) pour séparer une bande de feuilles servant de feuille de protection dans la direction du guidage en une première bande partielle (71) et en une deuxième bande partielle (72),
- au moins un élément de guidage (22a), qui est disposé dans la direction de guidage de la feuille après le dispositif de coupe (11) sur une arête externe de la première bande partielle (71), différente de l'arête de coupe, de telle sorte que cette arête externe est déviée et que l'arête de coupe d'une des deux bandes partielles (71, 72) est guidée en dessous de l'autre bande partielle.

10. Dispositif selon la revendication 9, qui comprend un deuxième élément de guidage (22) qui est disposé sur l'arête externe de la deuxième bande partielle (72), différente de l'arête de coupe, de telle sorte que l'arête de coupe de la deuxième bande partielle (72) est guidée le long de la direction de guidage ou dans la direction de la première bande partielle.

11. Dispositif selon l'une des revendications 9 ou 10, dans lequel le deuxième élément de guidage (22) est disposé par rapport à la direction de guidage de la bande de feuille en face du premier élément de guidage (22a).

12. Dispositif selon l'une des revendications 9 à 11, qui comprend au moins un autre élément de guidage (21a, 21) à travers lequel l'arête externe de la première et/ou de la deuxième bande partielle, différente de l'arête de coupe, et donc l'arête de coupe de chaque bande partielle sont guidées le long de la direction de guidage ou dans la direction de l'autre bande partielle.

13. Dispositif selon l'une des revendications 9 à 12, dans lequel l'élément de guidage (21, 22, 2 la, 22a) prend la forme d'un corps rotatif, à symétrie de rotation.

14. Dispositif selon l'une des revendications 9 à 13, dans lequel la déviation de l'arête externe de la première et/ou de la deuxième bande partielle (71, 72) par rapport à la direction de guidage ne dépasse pas respectivement 3,5 degrés et en particulier se situe dans la plage comprise entre 0,2 et 2,78 degrés et de préférence dans la plage comprise entre 0,2 et 0,7 degré.
